# EUROPEAN PATENT APPLICATION

(11) **EP 4 703 728 A1**
(43) Date of publication of application: **04.03.2026**
(21) Application number: 24796749.0
(22) Date of filing: 05.04.2024
(51) Int. Cl.: G01N 33/68, G01N 33/493, G01N 33/52

(54) **METHOD AND REAGENT FOR QUANTIFYING URINARY PROTEIN**

(30) Priority: 28.04.2023 JP 2023075293
(71) Applicant: Nitto Boseki Co., Ltd., Fukushima-shi, Fukushima 960-8161 (JP)
(72) Inventor: TOMARIGUCHI Natsuki, Koriyama-shi, Fukushima 963-8061 (JP); WAKABAYASHI Masayuki, Koriyama-shi, Fukushima 963-8061 (JP)
(74) Representative: Hoffmann Eitle
(86) International application number: PCT/JP2024/014047
(87) International publication number: WO 2024/224991

(57) **Abstract**

The present invention provides a method and a reagent for reducing hemolytic hemoglobin interference while correcting a difference in reactivity between urinary protein types.

Provided is a reagent for measuring protein in urine, comprising: a complex in which a metal is coordinated to a dye and the absorption wavelength of which shifts upon protein binding thereto; at least one anionic surfactant selected from a sulfonic acid-type anionic surfactant and a sulfuric acid ester-type anionic surfactant; and a polyoxyalkylene-type nonionic surfactant.

## Description

### TECHNICAL FIELD

The present invention relates to a method and a reagent for quantifying total protein in urine, and more specifically, to a method and a reagent for quantifying protein in urine using a complex in which a metal is coordinated to a dye.

### BACKGROUND ART

The total protein in urine is elevated in a kidney/urinary tract disease, and quantification of total protein in urine has been widely done for diagnosis of a kidney/urinary tract disease and confirmation of treatment effects on the disease. For quantification of total protein in urine in a clinical site, methods of measuring protein using a complex in which a metal is coordinated to a dye and an absorption wavelength of which shifts in the presence of the protein, such as a pyrogallol red-molybdenum method, have been widely used.

However, with the methods of quantifying protein using such a complex, some problems have been raised, including 1) a negative error due to a reactivity difference between protein species, and 2) a positive error due to hemolytic hemoglobin.

Regarding the negative error due to a reactivity difference between protein species, a method of correcting the reactivity difference between albumin and globulin by adding sodium dodecyl sulfate (SDS) has been reported (Non-Patent Literature 1). This report also teaches that the reactivity difference between albumin and globulin is eliminated by adjusting the concentration of SDS in the reagent to 25 mg/L. However, this report has not studied whether and how the addition of SDS affects substances coexisting in a specimen.

Hemolytic hemoglobin interference on clinical examination needs to be investigated from various viewpoints depending on reaction systems, and various methods of reducing the influence have been proposed (Patent Literatures 1 to 15). For example, in a method for quantifying bilirubin by two-point measurement, there is disclosed a method for reducing the influence of hemoglobin by suppressing change over time in the absorbance of hemoglobin with aliphatic higher sulfonic acid or a salt thereof, a sulfuric acid ester having an alkyl group with 11 to 16 carbon atoms, a primary amine with 11 to 16 carbon atoms or a salt thereof, or a quaternary ammonium salt (Patent Literature 8). For a method for reducing the influence of hemolytic hemoglobin or the like in measuring protein, a reaction(s) with a two-reagent system in which a copper ion-containing reagent with pH 10 to 13 is reacted with a specimen, followed by addition of an alkali solution such as lithium hydroxide has been proposed (Patent Literature 15). In addition, methods for reducing the influence of hemolytic hemoglobin as well as influences of other substances (for example, chyle and bilirubin) coexisting in a specimen have also been studied (Patent Literatures 2, 9, 10, and 12 to 15).

However, there is no report on how sodium dodecyl sulfate (SDS) affects hemolytic hemoglobin interference when the reactivity difference between albumin and globulin is corrected by adding SDS in a urine specimen containing hemolytic hemoglobin, and there is no report on a method for reducing hemolytic hemoglobin interference while correcting the reactivity difference between protein species in urine.

### CITATION LIST

### PATENT LITERATURE

PATENT LITERATURE 1: JP-A-2001-231596
PATENT LITERATURE 2: JP-A-2008-197077
PATENT LITERATURE 3: WO 2010/001619
PATENT LITERATURE 4: JP-A-2013-51907
PATENT LITERATURE 5: JP-A-09-84598
PATENT LITERATURE 6: JP-A-2000-189194
PATENT LITERATURE 7: JP-A-2015-29444
PATENT LITERATURE 8: JP-A-60-168050
PATENT LITERATURE 9: JP-A-2022-180380
PATENT LITERATURE 10: JP-A-2006-81471
PATENT LITERATURE 11: JP-A-2008-70346
PATENT LITERATURE 12: WO 2002/086151
PATENT LITERATURE 13: JP-A-2000-93200
PATENT LITERATURE 14: JP-A-2000-83698
PATENT LITERATURE 15: JP-A-10-19898

### NON-PATENT LITERATURE

NON-PATENT LITERATURE 1: CLIN. CHEM. Vol.35, No.11, 2233-2236 (1989)

### SUMMARY OF INVENTION

### TECHNICAL PROBLEM

Therefore, an object of the present invention is to provide a method and a reagent for reducing hemolytic hemoglobin interference while correcting a reactivity difference between protein species in urine.

### SOLUTION TO PROBLEM

The present inventors have confirmed a phenomenon in which, when a predetermined amount of sodium dodecyl sulfate (SDS) is added to a urine specimen containing hemolytic hemoglobin to correct a reactivity difference between albumin and globulin, SDS rather increases hemolytic hemoglobin interference. As a result of various studies on means for solving this problem, it has been found that this problem can also be overcome by reacting protein with a complex in the presence of a polyoxyalkylene-type nonionic surfactant together with a specific anionic surfactant such as SDS. That is, the present invention provides the following methods and reagents.
[1] A method for quantifying protein in urine using a complex in which a metal is coordinated to a dye and an absorption wavelength of which shifts upon binding to the protein, the method comprising:
   reacting the protein with the complex in presence of
   at least one anionic surfactant selected from a sulfonic acid-type anionic surfactant and a sulfuric acid ester-type anionic surfactant; and
   a polyoxyalkylene-type nonionic surfactant.
[2] A method for reducing hemolytic hemoglobin interference and reducing a reactivity difference between protein species in quantification of protein in urine using a complex in which a metal is coordinated to a dye and an absorption wavelength of which shifts upon binding to the protein, the method comprising:
   reacting the protein with the complex in presence of
   at least one anionic surfactant selected from a sulfonic acid-type anionic surfactant and a sulfuric acid ester-type anionic surfactant; and
   a polyoxyalkylene-type nonionic surfactant.
[3] The method according to [1] or [2], wherein
   the sulfonic acid-type anionic surfactant is a sulfonate represented by Formula (1):

      R¹-SO₃X¹··· (1)
   wherein
   R¹ is selected from the group consisting of:
      (1) a hydrocarbon group having 8 to 18 carbon atoms,
      (2) a monocyclic or bicyclic aromatic hydrocarbon substituted with a hydrocarbon group having 8 to 18 carbon atoms,
      (3) a succinic acid residue of Formula (2), a (di)ester of the succinic acid residue, or a salt of the succinic acid residue or the (di)ester: 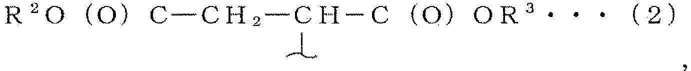 wherein R² and R³ each independently represent hydrogen, a hydrocarbon group having 8 to 18 carbon atoms, or an alkali metal,
      (4) a succinic acid monoamide residue of Formula (3), an ester of the succinic acid monoamide residue, or a salt of the succinic acid monoamide residue or the ester: 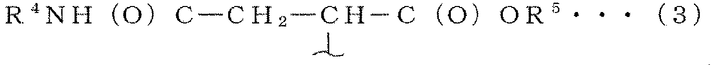 wherein R⁴ and R⁵ each independently represent hydrogen, a hydrocarbon group having 8 to 18 carbon atoms, or an alkali metal, and
      (5) a sulfonate of a hydrocarbon group-substituted diphenyl ether residue of Formula (4): 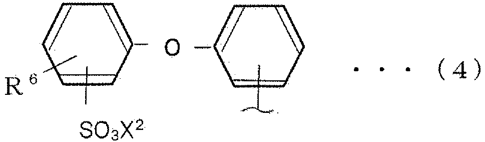
         wherein R⁶ is a hydrocarbon group having 8 to 18 carbon atoms, and X² is an alkali metal, ammonium, or alkanolamine, and
         X¹ is an alkali metal, ammonium, or alkanolamine.
[4] The method according to [3], wherein R¹ is (1) an alkyl group or alkenyl group having 8 to 18 carbon atoms or (2) a monocyclic or bicyclic aromatic hydrocarbon substituted with an alkyl group or alkenyl group having 8 to 18 carbon atoms.
[5] The method according to [4], wherein the sulfonic acid-type anionic surfactant is an alkane sulfonate having 11 to 16 carbon atoms or a linear alkylbenzene sulfonate having 10 to 14 carbon atoms.
[6] The method according to [1] or [2], wherein the sulfuric acid ester-type anionic surfactant is a sulfuric acid ester represented by Formula (5) or (6):

   R⁷O-SO₃X³··· (5)

   wherein R⁷ is a hydrocarbon group having 8 to 18 carbon atoms or a monocyclic or bicyclic aromatic hydrocarbon substituted with a hydrocarbon group having 8 to 18 carbon atoms, and X³ is an alkali metal, ammonium, or alkanolamine, or

      R⁸O-(CHR⁹CHR¹⁰O)ₙ-SO₃X⁴··· (6)
   wherein R⁸ is a hydrocarbon group having 8 to 18 carbon atoms, R⁹ and R¹⁰ are independently hydrogen or an alkyl group having 1 to 3 carbon atoms, n is 1 to 4, and X⁴ is an alkali metal, ammonium, or alkanolamine.
[7] The method according to [6], wherein the sulfuric acid ester-type anionic surfactant is an alkyl sulfate having 11 to 16 carbon atoms.
[8] The method according to any one of [1] to [7], wherein the polyoxyalkylene-type nonionic surfactant is a polyoxyalkylene compound of Formula (7) or (8):

   R¹¹-Y¹-((CH₂)ₘO)ₙ-R¹²··· (7)

   wherein Y¹ is O, C(O)O, or C(O)NH, R¹¹ is a chain hydrocarbon group having 8 to 22 carbon atoms, m is an integer of 1 to 4, n is an integer of 2 to 35, and R¹² is hydrogen or an alkyl group having 1 to 3 carbon atoms,
      [Chem. 8]

      R¹³-Y²-((CH₂)ₘ₁O)n₁-((CH₂)ₘ₂O)ₙ₂-((CH₂)ₘ₃O)ₙ₃-R¹⁴··· (8)
   wherein Y² is O, C(O)O, or C(O)NH, m1 and m3 are each independently an integer of 1 to 4, m2 is an integer of 2 to 4, m1 and/or m3 is different from m2, n1, n2, and n3 are each independently an integer of 2 to 200, R¹³ is hydrogen or a chain hydrocarbon group having 1 to 30 carbon atoms, and R¹⁴ is hydrogen or an alkyl group having 1 to 3 carbon atoms.
[9] The method according to [8], wherein the polyoxyalkylene-type nonionic surfactant is a polyoxyalkylene compound of Formula (7) wherein Y¹ is O or C(O)O, R¹¹ is a linear alkyl group or alkenyl group having 11 to 20 carbon atoms, m is 2, n is an integer of 20 to 35, and R¹² is hydrogen.
[10] The method according to any one of [1] to [9], wherein the nonionic surfactant in a reaction solution has a concentration of 0.0015 to 0.030% by mass, and the anionic surfactant in the reaction solution has a concentration of 0.001% by mass to 0.0035% by mass.
[11] The method according to [10], wherein the anionic surfactant in the reaction solution has a concentration of 0.0015 to 0.0030% by mass.
[12] The method according to [10] or [11], wherein the nonionic surfactant in the reaction solution has a concentration of 0.0025 to 0.024% by mass.
[13] The method according to any one of [1] to [12], wherein the dye is pyrogallol red or pyrocatechol violet, and the metal is molybdenum, tin, or iron.
[14] A reagent for measuring protein in urine comprising:
   a complex in which a metal is coordinated to a dye and an absorption wavelength of which shifts upon binding to the protein;
   at least one anionic surfactant selected from a sulfonic acid-type anionic surfactant and a sulfuric acid ester-type anionic surfactant; and
   a polyoxyalkylene-type nonionic surfactant.
[15] The reagent for measuring protein in urine according to [14], wherein
   the sulfonic acid-type anionic surfactant is a sulfonate represented by Formula (1):

      R¹-SO₃X¹··· (1)
   wherein
   R¹ is selected from the group consisting of:
      (1) a hydrocarbon group having 8 to 18 carbon atoms,
      (2) a monocyclic or bicyclic aromatic hydrocarbon substituted with a hydrocarbon group having 8 to 18 carbon atoms,
      (3) a succinic acid residue of Formula (2), a (di)ester of the succinic acid residue, or a salt of the succinic acid residue or the (di)ester: 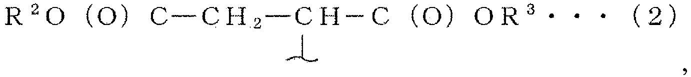 wherein R² and R³ each independently represent hydrogen, a hydrocarbon group having 8 to 18 carbon atoms, or an alkali metal,
      (4) a succinic acid monoamide residue of Formula (3), an ester of the succinic acid monoamide residue, or a salt of the succinic acid monoamide residue or the ester: 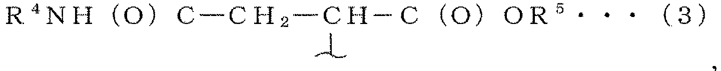 wherein R⁴ and R⁵ each independently represent hydrogen, a hydrocarbon group having 8 to 18 carbon atoms, or an alkali metal, and
      (5) a sulfonate of a hydrocarbon group-substituted diphenyl ether residue of Formula (4): 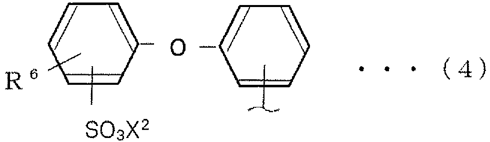
         wherein R⁶ is a hydrocarbon group having 8 to 18 carbon atoms, and X² is an alkali metal, ammonium, or alkanolamine, and
         X¹ is an alkali metal, ammonium, or alkanolamine.
[16] The reagent for measuring protein in urine according to [15], wherein R¹ is (1) an alkyl group or alkenyl group having 8 to 18 carbon atoms, or (2) a monocyclic or bicyclic aromatic hydrocarbon substituted with an alkyl group or alkenyl group having 8 to 18 carbon atoms.
[17] The reagent for measuring protein in urine according to [16], wherein the sulfonic acid-type anionic surfactant is an alkane sulfonate having 11 to 16 carbon atoms or a linear alkylbenzene sulfonate having 10 to 14 carbon atoms.
[18] The reagent for measuring protein in urine according to [14], wherein the sulfuric acid ester-type anionic surfactant is a sulfuric acid ester represented by Formula (5) or (6):

   R⁷O-SO₃X³··· (5)

   wherein R⁷ is a hydrocarbon group having 8 to 18 carbon atoms, or a monocyclic or bicyclic aromatic hydrocarbon substituted with a hydrocarbon group having 8 to 18 carbon atoms, and X³ is an alkali metal, ammonium, or alkanolamine, or

      R⁸O-(CHR⁹CHR¹⁰O)ₙ-SO₃X⁴··· (6)
   wherein R⁸ is a hydrocarbon group having 8 to 18 carbon atoms, R⁹ and R¹⁰ are independently hydrogen or an alkyl group having 1 to 3 carbon atoms, n is 1 to 4, and X⁴ is an alkali metal, ammonium, or alkanolamine.
[19] The reagent for measuring protein in urine according to [18], wherein the sulfuric acid ester-type anionic surfactant is an alkyl sulfate having 11 to 16 carbon atoms.
[20] The reagent for measuring protein in urine according to any one of [14] to [19], wherein the polyoxyalkylene-type nonionic surfactant is a polyoxyalkylene compound of Formula (7) or (8):

   R¹¹-Y¹-((CH₂)ₘO)ₙ-R¹²··· (7)

   wherein Y¹ is O, C(O)O, or C(O)NH, R¹¹ is a chain hydrocarbon group having 8 to 22 carbon atoms, m is an integer of 1 to 4, n is an integer of 2 to 35, and R¹² is hydrogen or an alkyl group having 1 to 3 carbon atoms, or

      R¹³-Y²-((CH₂)ₘ₁O)ₙ₁-((CH₂)ₘ₂O)ₙ₂-((CH₂)ₘ₃O)ₙ₃-R¹⁴··· (8)
   wherein Y² is O, C(O)O, or C(O)NH, m1 and m3 are each independently an integer of 1 to 4, m2 is an integer of 2 to 4, m1 and/or m3 is different from m2, n1, n2, and n3 are each independently an integer of 2 to 200, R¹³ is hydrogen or a chain hydrocarbon group having 1 to 30 carbon atoms, and R¹⁴ is hydrogen or an alkyl group having 1 to 3 carbon atoms.
[21] The reagent for measuring protein in urine according to [20], wherein the polyoxyalkylene-type nonionic surfactant is a polyoxyalkylene compound of Formula (7) wherein Y¹ is O or C(O)O, R¹¹ is a linear alkyl group or alkenyl group having 11 to 20 carbon atoms, m is 2, n is an integer of 20 to 35, and R¹² is hydrogen.
[22] The reagent for measuring protein in urine according to any one of [14] to [21], wherein the nonionic surfactant has a concentration of 0.0015 to 0.030% by mass, and the anionic surfactant has a concentration of 0.001% by mass to 0.0035% by mass.
[23] The reagent for measuring protein in urine according to [22], wherein the anionic surfactant has a concentration of 0.0015 to 0.0030% by mass.
[24] The reagent for measuring protein in urine according to [22] or [23], wherein the nonionic surfactant has a concentration of 0.0025 to 0.024% by mass.
[25] The reagent for measuring protein in urine according to any one of [14] to [24], wherein the dye is pyrogallol red or pyrocatechol violet, and the metal is molybdenum, tin, or iron.
[26] The reagent for measuring protein in urine according to any one of [14] to [25], which is a single-solution type reagent for measurement.
[27] A method for producing a reagent for measuring protein in urine, comprising dissolving, in a buffer solution,
   a dye selected from pyrocatechol violet, pyrogallol red, brompyrogallol red, o-hydroxyhydroquinonephthalein, and gallein;
   an oxyacid salt, a halide, a complex salt, or an organic or inorganic acid salt of a metal selected from molybdenum, tin, and iron;
   at least one anionic surfactant selected from a sulfonic acid-type anionic surfactant and a sulfuric acid ester-type anionic surfactant; and
   a polyoxyalkylene-type nonionic surfactant.

### ADVANTAGEOUS EFFECTS OF INVENTION

In the quantification of total protein in urine using a complex in which a metal is coordinated to a dye, such as a pyrogallol red-molybdenum complex, hemolytic hemoglobin interference can be reduced while correcting a reactivity difference between protein species in urine.

### DESCRIPTION OF EMBODIMENTS

An embodiment of the present invention will be described in detail. However, the present invention should not be understood as being limited to the following embodiments.

A method for quantifying protein according to the present invention is characterized by, in quantification of protein in urine using a complex in which a metal is coordinated to a dye (hereinafter, sometimes abbreviated as a "metal-dye complex" or a "complex"), reacting the complex with the protein in the presence of a specific anionic surfactant and a specific nonionic surfactant. A reagent for measuring protein in urine according to the present invention is characterized by combining a metal-dye complex with a specific anionic surfactant and a specific nonionic surfactant in such a manner that at least both of the complex and the surfactants coexist in a reaction solution.

A dye constituting a metal-dye complex may be any dye that coordinates with a metal to form a complex and causes the complex to occur a shift of the absorption wavelength upon binding of protein to the complex. Examples of the dye include pyrocatechol violet, pyrogallol red, brompyrogallol red, o-hydroxyhydroquinonephthalein, and gallein. A metal constituting a metal-dye complex may also be any metal that is coordinated to the dye to form a complex, and causes the complex to occur a shift of the absorption wavelength upon binding of protein to the complex. Examples of the metal include molybdenum, tin, iron, and aluminum. Specific examples of a metal-dye complex include a pyrocatechol violet-tin (IV) complex, a pyrogallol red-molybdenum complex, a brompyrogallol red-molybdenum complex, an o-hydroxyhydroquinonephthalein-iron (III) complex, an o-hydroxyhydroquinonephthaleinaluminum (III), and a gallein-molybdenum complex.

The concentration of a metal-dye complex in a reaction solution is set to be sufficient for an assumed protein concentration in a sample, and the concentration of a metal-dye complex in a reaction solution may be usually 0.030 to 0.090 mM, and is preferably 0.037 to 0.057 mM. The concentration of a metal-dye complex in a measurement reagent is designed to achieve such a concentration of a metal-dye complex in a reaction solution, and the concentration of a metal-dye complex in a measurement reagent is usually adjusted to 0.030 to 0.090 mM, and preferably adjusted to 0.037 to 0.057 mM.

A reagent containing a metal-dye complex is prepared by dissolving a dye and a metal as described above in a buffer solution adjusted to a pH predetermined depending on the type of a complex. The metal is usually mixed with the dye in the form of an oxyacid salt, a halide, a complex salt, or an organic or inorganic acid salt. Examples of such a form include molybdates (alkali metal salts, ammonium salts, and the like), stannates (alkali metal salts, ammonium salts, and the like), tin chloride, tin sulfate, ferrates (alkali metal salts, ammonium salts, and the like), and iron chloride. In preparing the reagent, the amounts of a dye and a metal to be added to a buffer solution are determined depending on the concentration of a complex to be formed. The amount of a dye added may be usually 0.040 to 0.180 mM, and is preferably 0.054 to 0.081 mM, in a measurement reagent. The amount of a metal added may be usually 0.030 to 0.090 mM, and is preferably 0.037 to 0.057 mM, in a measurement reagent.

A method according to the present invention is performed by bringing a metal-dye complex into contact with protein in a sample in the presence of at least one anionic surfactant selected from a sulfonic acid-type anionic surfactant and a sulfuric acid ester-type anionic surfactant, and a polyoxyalkylene-type nonionic surfactant. The presence of these surfactants reduces hemolytic hemoglobin interference while correcting a reactivity difference between protein species in urine.

As used herein, the "sulfonic acid-type anionic surfactant" means an anionic surfactant having one or more sulfonic acid groups or salts thereof.

In a preferred embodiment, the sulfonic acid-type anionic surfactant is a sulfonate represented by Formula (1):

R¹-SO₃X¹··· (1)

wherein
R¹ is selected from the group consisting of:
   (1) a hydrocarbon group, preferably a hydrocarbon group having 8 to 18 carbon atoms, more preferably an alkyl group or alkenyl group having 8 to 18 carbon atoms, and particularly preferably a linear alkyl group having 11 to 16 carbon atoms,
   (2) a monocyclic or bicyclic aromatic hydrocarbon substituted with a hydrocarbon group, preferably a monocyclic or bicyclic aromatic hydrocarbon substituted with a hydrocarbon group having 8 to 18 carbon atoms, more preferably a 5- or 6-membered monocyclic aromatic hydrocarbon or a 10 to 12-membered bicyclic aromatic hydrocarbon substituted with a hydrocarbon group having 8 to 18 carbon atoms, still more preferably a 5- or 6-membered monocyclic aromatic hydrocarbon or a 10 to 12-membered bicyclic aromatic hydrocarbon substituted with an alkyl group or alkenyl group having 8 to 18 carbon atoms, and particularly preferably a benzene ring substituted with a linear alkyl group having 10 to 14 carbon atoms: Meanwhile, the bonding between a hydrocarbon group and an aromatic ring can be at any chemically possible position,
   (3) a succinic acid residue of Formula (2), a (di)ester thereof, or a salt of the residue or ester: 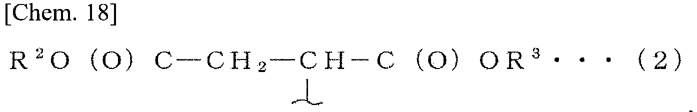 wherein R² and R³ each independently represent hydrogen, a hydrocarbon group or an alkali metal, preferably a hydrocarbon group having 8 to 18 carbon atoms or Na, and more preferably a linear alkyl group having 8 to 16 carbon atoms,
   (4) a succinic acid monoamide residue of Formula (3), an ester thereof, or a salt of the residue or ester: 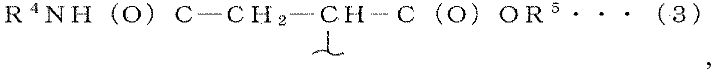 wherein R⁴ and R⁵ each independently represent hydrogen, a hydrocarbon group or an alkali metal, preferably a hydrocarbon group having 8 to 18 carbon atoms or Na, and more preferably a linear alkyl group having 11 to 16 carbon atoms, and
   (5) a sulfonate of a hydrocarbon group-substituted diphenyl ether residue of Formula (4): 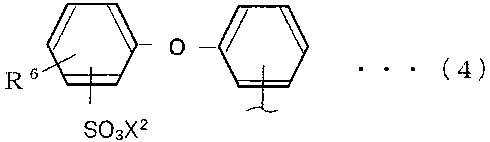
wherein R⁶ is a hydrocarbon group having 8 to 18 carbon atoms, preferably an alkyl group or alkenyl group having 8 to 18 carbon atoms, and particularly preferably a linear alkyl group having 11 to 16 carbon atoms, and X² is an alkali metal, ammonium, or alkanolamine (preferably, an alkanolamine having 1 to 3 carbon atoms), and preferably Na, and
X¹ is an alkali metal, ammonium, or alkanolamine (preferably, an alkanolamine having 1 to 3 carbon atoms), and preferably Na.

A more preferable sulfonic acid-type anionic surfactant is an alkane sulfonate having 11 to 16 carbon atoms or a linear alkylbenzene sulfonate having 10 to 14 carbon atoms, and a Na salt thereof is particularly preferable.

As used herein, the "sulfuric acid ester-type anionic surfactant" is an anionic surfactant having one or more structures represented by a formula: -O-SO₃⁻, and examples thereof include alkyl sulfuric acid ester, polyoxyethylene alkyl ether sulfuric acid ester, and salts thereof.

In a preferred embodiment, the sulfuric acid ester-type anionic surfactant is a sulfuric acid ester salt represented by Formula (5) or (6):

R⁷O-SO₃X³··· (5)

wherein R⁷ is a hydrocarbon group having 8 to 18 carbon atoms or a monocyclic or bicyclic aromatic hydrocarbon substituted with a hydrocarbon group having 8 to 18 carbon atoms; the hydrocarbon group having 8 to 18 carbon atoms is preferably an alkyl group or alkenyl group having 8 to 18 carbon atoms, and more preferably an alkyl group having 11 to 16 carbon atoms; the monocyclic or bicyclic aromatic hydrocarbon substituted with a hydrocarbon group having 8 to 18 carbon atoms is preferably a 5- or 6-membered monocyclic aromatic hydrocarbon or a 10 to 12-membered bicyclic aromatic hydrocarbon substituted with a hydrocarbon group having 8 to 18 carbon atoms, more preferably a 5- or 6-membered monocyclic aromatic hydrocarbon or a 10 to 12-membered bicyclic aromatic hydrocarbon substituted with an alkyl group or alkenyl group having 8 to 18 carbon atoms, and particularly preferably a benzene ring substituted with a linear alkyl group having 10 to 14 carbon atoms (the bonding between the hydrocarbon and the aromatic ring can be at any chemically possible position); and X³ is an alkali metal, ammonium, or alkanolamine (preferably, an alkanolamine having 1 to 3 carbon atoms), and preferably Na, or

   R⁸O-(CHR⁹CHR¹⁰O)ₙ-SO₃X⁴··· (6)
wherein R⁸ is a hydrocarbon group having 8 to 18 carbon atoms (including an alkyl group and an alkenyl group), and preferably an alkyl group having 11 to 16 carbon atoms, R⁹ and R¹⁰ are independently hydrogen or an alkyl group having 1 to 3 carbon atoms, and preferably hydrogen, n is 1 to 10, and preferably 1 to 4, and X⁴ is an alkali metal, ammonium, or alkanolamine (preferably, an alkanolamine having 1 to 3 carbon atoms), and preferably Na.

A more preferable sulfuric acid ester-type anionic surfactant is an alkyl sulfate having 11 to 16 carbon atoms, and a Na salt thereof is particularly preferable.

The concentration of a sulfuric acid ester-type anionic surfactant or a sulfonic acid-type anionic surfactant in a reaction solution can be determined depending on measurement parameters to be applied such that the sulfuric acid ester-type anionic surfactant or the sulfonic acid-type anionic surfactant can work with a polyoxyalkylene-type nonionic surfactant to reduce hemolytic hemoglobin interference while correcting a reactivity difference between protein species in urine. The concentration is preferably 0.001% by mass to 0.004% by mass, more preferably 0.001% by mass to 0.0035% by mass, still more preferably 0.0015% by mass to 0.0030% by mass, and particularly preferably 0.0015% by mass to 0.0025% by mass. The concentration of a sulfuric acid ester-type anionic surfactant or a sulfonic acid-type anionic surfactant in a measurement reagent can be designed to achieve the desired concentration in a reaction solution. The concentration is preferably 0.001% by mass to 0.004% by mass, more preferably 0.001% by mass to 0.0035% by mass, more preferably 0.0015% by mass to 0.003% by mass, and particularly preferably 0.0015% by mass to 0.0025% by mass.

As used herein, a "polyoxyalkylene-type nonionic surfactant" is an anionic surfactant having a structure of a formula: R-Y-(alkylene-O)ₙ- wherein R is a chain hydrocarbon group, Y is O, C(O)O, or C(O)NH, and n is an integer of 1 or more and 50 or less.

In a preferred embodiment, the polyoxyalkylene-type nonionic surfactant is a polyoxyalkylene compound represented by Formula (7) or (8).

R¹¹-Y¹-((CH₂)ₘO)ₙ-R¹²··· (7)

wherein, Y¹ is O, C(O)O, or C(O)NH, and preferably O or C(O)O; R¹¹ is a chain hydrocarbon group having 8 to 22 carbon atoms, preferably an alkyl group or alkenyl group having 8 to 22 carbon atoms, and more preferably a linear alkyl group or alkenyl group having 11 to 20 carbon atoms; m is an integer of 1 to 4, and preferably 2; n is an integer of 2 to 35, preferably an integer of 15 to 35, and more preferably an integer of 20 to 35; and R¹² is hydrogen or an alkyl group having 1 to 3 carbon atoms, and preferably hydrogen.

   R¹³-Y²-((CH₂)ₘ₁O)ₙ₁-((CH₂)m₂O)ₙ₂-((CH₂)ₘ₃O)ₙ₃-R¹⁴··· (8)
wherein, Y² is O, C(O)O or C(O)NH, preferably O or C(O)O, and more preferably O; m1 and m3 are each independently an integer of 1 to 4, and preferably 2; m2 is an integer of 2 to 4, and preferably 3; m1 or m3 is different from m2, and preferably m1 and m3 are different from m2; n1, n2, and n3 are each independently an integer of 2 to 200, preferably an integer of 2 to 100, and more preferably an integer of 2 to 70; R¹³ is hydrogen or a chain hydrocarbon group having 1 to 30 carbon atoms, preferably a chain hydrocarbon group having 8 to 22 carbon atoms, more preferably an alkyl group or alkenyl group having 8 to 22 carbon atoms, and particularly preferably an alkyl group or alkenyl group having 11 to 20 carbon atoms; and R¹⁴ is hydrogen or an alkyl group having 1 to 3 carbon atoms, and preferably hydrogen.

As a polyoxyalkylene-type nonionic surfactant, a surfactant having an HLB value of 13 to 19 is preferable, and a surfactant having an HLB value of 15 to 19 is more preferable. As used herein, the HLB value refers to a value obtained by Griffin's equation (Surfactant Handbook, Kougakutosho Ltd., published in 1987, pp. 234-235).

The concentration of a polyoxyalkylene-type nonionic surfactant in a reaction solution can be determined depending on measurement parameters to be applied such that the polyoxyalkylene-type nonionic surfactant can work with a sulfuric acid ester-type anionic surfactant or a sulfonic acid-type anionic surfactant to reduce hemolytic hemoglobin interference while correcting a reactivity difference between protein species in urine. The concentration is preferably 0.0015% by mass to 0.03% by mass, more preferably 0.0025% by mass to 0.024% by mass, more preferably 0.003% by mass to 0.012% by mass, and particularly preferably 0.004% by mass to 0.008% by mass. The concentration of a polyoxyalkylene-type nonionic surfactant in a measurement reagent can be designed to achieve the desired concentration in a reaction solution. The concentration is preferably 0.0015% by mass to 0.03% by mass, more preferably 0.0025% by mass to 0.024% by mass, still more preferably 0.003% by mass to 0.012% by mass, and particularly preferably 0.004% by mass to 0.008% by mass.

In the present invention, the anionic surfactant and the nonionic surfactant described above only need to be present during a reaction between a metal-dye complex and protein. Therefore, these surfactants may be added to a reagent solution containing the metal-dye complex, they may be added to a specimen such as urine, or they may be added at the time of mixing the measurement reagent and the specimen. From the viewpoint of practical use, it is convenient to add these surfactants in advance to a reagent containing a metal-dye complex. In the quantification of protein in urine, a single-solution type reagent is often used. In this case, these surfactants are contained together with other components.

In a method and a reagent according to the present invention, components other than the metal-dye complex, the anionic surfactant, and the nonionic surfactant described above are not particularly limited, and other components used in a reagent and a method for quantifying protein in urine by a reaction between a metal-dye complex and the protein can be contained. For example, there are other components that reduce the influence of hemolytic hemoglobin, components that reduce coexisting substances other than hemolytic hemoglobin, and the like, and these are disclosed in, for example, Patent Literatures 1 to 18,the contents of which are incorporated herein by reference.

Examples of components that reduce the coexisting substances include a polyhydric alcohol and a chelating agent.

Examples of the polyhydric alcohol include glycols and sugar alcohols, and specific examples thereof include mannitol, sorbitol, dulcitol, glycerol, and polyglycerol. These polyhydric alcohols can be used singly or in combination of two or more.

Examples of the chelating agent include ethylenediaminetetraacetic acid (EDTA), hydroxyethylethylenediaminetriacetic acid (EDTA-OH), ethylenediaminediacetic acid (EDDA), iminodiacetic acid (IDA), nitrilopropionic acid (NTP), nitrilotriacetic acid (NTA), hydroxyethyliminodiacetic acid (HIDA), citric acid, tartaric acid, oxalic acid, 1-hydroxyethane-1,1-diphosphonic acid, pyrophosphoric acid, hexametaphosphoric acid, tripolyphosphoric acid, metaphosphoric acid, and salts thereof. These chelating agents can be used singly or in combination of two or more. Examples of the salt include alkali metal salts such as sodium, potassium, and lithium, and ammonium salts.

A measurement reagent according to the present invention can be prepared by dissolving each component described above in a buffer solution adjusted to a predetermined pH. The buffer solution is selected depending on metal-dye complexes to be used, and examples thereof include a glycine buffer solution, a maleic acid buffer solution, a succinic acid buffer solution, a citric acid buffer solution, an oxalic acid buffer solution, and a phosphoric acid buffer solution. The pH of a measurement reagent (buffer solution) is also selected depending on metal-dye complexes to be used such that the complex formation and the absorption wavelength shift appropriately occur. For example, in the case of a pyrogallol-molybdenum complex, selected is pH 2 to 2.5, and preferably pH 2.2.

Quantification of protein in urine according to the present invention can be performed in the same manner as in conventional methods using a metal-dye complex, except that a reaction between a metal-dye complex and protein is performed in the presence of an anionic surfactant and a nonionic surfactant as described above. As an example thereof, a measurement reagent containing the above-described components and a specimen for quantifying the protein concentration are mixed and reacted at a constant temperature of room temperature to 37°C for a constant time (for example, about 3 to 15 minutes). A specimen for quantifying the protein concentration may be appropriately diluted as necessary, and the measurement may be performed on the diluted sample. The absorption wavelength of a complex shifts when protein binds to the metal-dye complex. Therefore, the absorbance is measured by means of using a wavelength near the maximum absorption wavelength after the shift as the main wavelength, and optionally further selecting a secondary wavelength, and. On the other hand, a standard sample with a known protein concentration is used in place of the specimen, and the absorbance is measured in the same manner to create a calibration curve. The protein concentration in the specimen can be determined by comparing the absorbance obtained in the specimen with the calibration curve.

A method according to the present invention can be easily performed in a short time by a colorimetric method and is suitable for measurement using an automatic analyzer. The method can also be applied to the measurement of protein by a test strip method.

### [Examples]

Hereinafter, the present invention will be described more specifically with reference to examples, but the present invention is not limited thereto.

### 1. Evaluation Methods

In order to evaluate the effect of correcting a reactivity difference between protein species in urine and the effect of reducing hemolytic hemoglobin interference by reagents according to the present invention, the following tests were performed on the reagents of Examples and Comparative examples described below.

### 1-1. Test for Evaluating Reactivity Difference Between Protein Species in Urine

The measurement reagent of each of Examples and Comparative Examples was used to quantify the total protein concentration of an albumin solution having a known concentration and the total protein concentration of a γ-globulin solution having a known concentration, and the analytical value was compared with the known concentration to evaluate reactivity to both protein species.

### (1) Preparation of Human Serum Albumin Solution and Human Serum γ-Globulin Solution

Human serum albumin (manufactured by SCRIPPS LABORATORIES) and human serum γ-globulin (manufactured by SIGMA) each were weighed and dissolved in ion-exchanged water to prepare solutions at concentrations of 0 mg/dl, 120 mg/dl, and 600 mg/dl.

### (2) Quantification of Total Protein

The total protein of a human serum albumin solution at each concentration and the total protein of human serum γ-globulin at each concentration were quantified using the measurement reagent of each of Examples and Comparative Examples by Hitachi 7180 automatic analyzer (Hitachi, Ltd.). Human serum albumin (manufactured by SCRIPPS LABORATORIES) was weighed, dissolved in ion-exchanged water, adjusted to a concentration of 100 mg/dl, and used as a calibrator. The measurement was performed using the following measurement parameters.

| | |
|---|---|
| Amount of specimen | 2.0 µL |
| Amount of first reagent | 250 µL |
| Measurement wavelength (main)/(sub) | 600 nm/660 nm |
| Analytical method one | point end assay |
| Photometric measurement point | 34 |

### 1-2. Effect of Reducing Hemolytic Hemoglobin Interference

The measurement reagent of each of Examples and Comparative Examples was used to quantify the total protein concentration of a urine sample containing hemolytic hemoglobin at a predetermined concentration, and the influence of hemolytic hemoglobin on the analytical value was evaluated.

### (1) Preparation of Hemolytic Hemoglobin Solution

A blank (hemoglobin concentration: 0 mg/dL) and a 1000 mg/dL hemolytic hemoglobin solution contained in Interference Check A Plus (manufactured by Sysmex Corporation) were each diluted 10 times with a urine control sample (Liquichek 2, manufactured by Bio-Rad Laboratories, Inc.) to prepare urine-based samples having hemoglobin concentrations of 0 mg/dL and 100 mg/dL.

### (2) Quantification of Total Protein

The total protein in each of the solutions was quantified using the same apparatus and under the same conditions as those for the quantification of the total protein performed for evaluating a reactivity difference between protein species in urine.

### 2. Study on Kind of Surfactants

### 2-1. Preparation of Reagents

### [Example 1]

A measurement reagent containing a complex of pyrogallol red and molybdenum was prepared by adding 0.068 mM pyrogallol red, 0.047 mM ammonium molybdate, 12.65 mM sodium benzoate, 13.47 mM D-mannitol, 0.81 mM sulfanilic acid, 0.04% by mass EDTA-OH, 0.002% by mass Na dodecyl sulfate (SDS), and 0.008% by mass Brij 58 to a 100 mM glycine buffer solution, mixing them, and adjusting the pH to 2.2.
Na dodecyl sulfate (SDS) 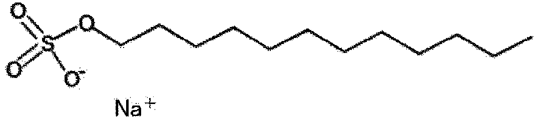
Brij 58
   Polyoxyethylene(20) cetyl ether

   CH₃(CH₂)₁₅O((CH₂)₂O)₂₀H

### [Comparative Example 1]

A measurement reagent was prepared in the same manner as in Example 1 except that neither Na dodecyl sulfate (SDS) nor Brij 58 was added.

### [Comparative Example 2]

A measurement reagent was prepared in the same manner as in Example 1 except that Brij 58 was not added.

### [Comparative Example 3]

A measurement reagent was prepared in the same manner as in Example 1 except that Na dodecyl sulfate (SDS) was not added.

### [Example 2] and [Comparative Example 4]

Measurement reagents were each prepared in the same manner as in Example 1 except that instead of 0.002% by mass Na dodecyl sulfate (SDS), sodium linear alkylbenzene sulfonate or sodium cholate was added at the same concentration.

### Sodium linear alkylbenzene sulfonate

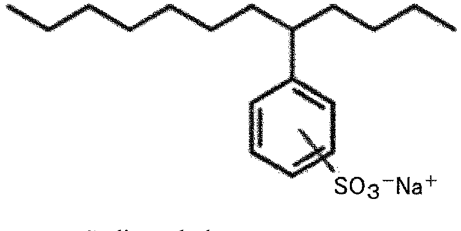

Sodium cholate

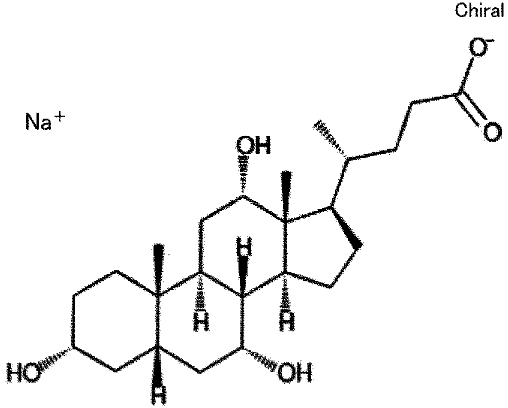

### [Comparative Examples 5 to 7]

Measurement reagents were each prepared in the same manner as in Example 1 except that instead of 0.002% by mass Na dodecyl sulfate (SDS), n-octylamine hydrochloride, benzylcetyldimethylammonium chloride, or lauryldimethylaminoacetic acid betaine was added at the same concentration.

### n-Octylamine hydrochloride

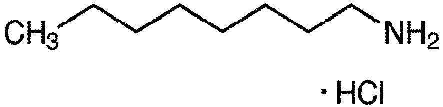

### Benzylcetyldimethylammonium chloride

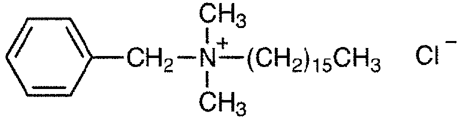

### Lauryldimethylaminoacetic acid betaine

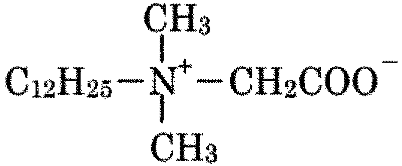

### [Examples 3 to 5]

Measurement reagents were each prepared in the same manner as in Example 1 except that instead of 0.008% by mass Brij 58, Brij 35, NONION E-230, or NONION S15.4 was added at the same concentration.
Brij 35
   Polyoxyethylene(23) lauryl ether

   CH₃(CH₂)₁₁O((CH₂)₂O)₂₃H
NONION E-230
   Polyoxyethylene(30) oleyl ether

   CH₃(CH₂)₇CH=CH(CH₂)₈O((CH₂)₂O)₃₀H
NONION S15.4
   Polyethylene glycol(35) monostearate

   CH₃(CH₂)₁₆C(O)O((CH₂)₂O)₃₅H

### [Comparative Examples 8 to 10]

Measurement reagents were each prepared in the same manner as in Example 1 except that instead of 0.008% by mass Brij 58, Triton X-100, BPSH-25, or Tween 20 was added at the same concentration.

### Triton X-100

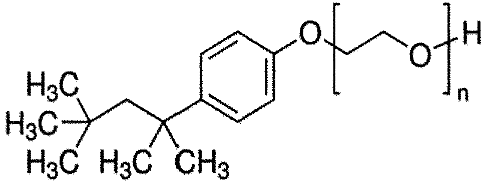

### BPSH-25

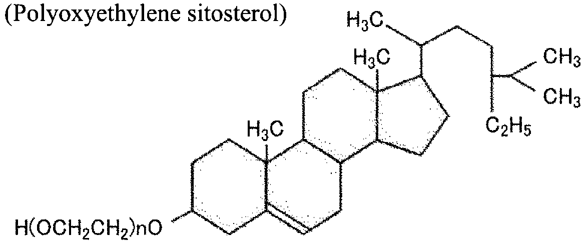

### Tween 20

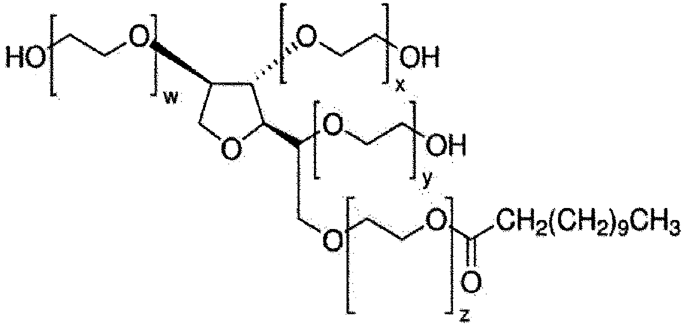

The compositions of the measurement reagents of Examples 1 to 5 and Comparative Examples 1 to 10 are summarized below.

**[Table 1]**

| | Comparative Example 1 | Comparative Example 2 | Comparative Example 3 | Example 1 | Example 2 | Comparative Example 4 |
|---|---|---|---|---|---|---|
| Glycine | 100 mM | 100 mM | 100 mM | 100 mM | 100 mM | 100 mM |
| Pyrogallol red | 0.068 mM | 0.068 mM | 0.068 mM | 0.068 mM | 0.068 mM | 0.068 mM |
| Ammonium molybdate | 0.047 mM | 0.047 mM | 0.047 mM | 0.047 mM | 0.047 mM | 0.047 mM |
| D-Mannitol | 13.47 mM | 13.47 mM | 13.47 mM | 13.47 mM | 13.47 mM | 13.47 mM |
| Sulfanilic acid | 0.81 mM | 0.81 mM | 0.81 mM | 0.81 mM | 0.81 mM | 0.81 mM |
| Sodium benzoate | 12.65 mM | 12.65 mM | 12.65 mM | 12.65 mM | 12.65 mM | 12.65 mM |
| EDTA-OH | 0.04% | 0.04% | 0.04% | 0.04% | 0.04% | 0.04% |
| SDS | - | 0.002% | - | 0.002% | - | - |
| Sodium linear alkylbenzene sulfonate | - | - | - | - | 0.002% | - |
| Sodium cholate | - | | - | - | - | 0.002% |
| Brij58 | - | - | 0.008% | 0.008% | 0.008% | 0.008% |

**[Table 2]**

| | Comparative Example 5 | Comparative Example 6 | Comparative Example 7 |
|---|---|---|---|
| Glycine | 100 mM | 100 mM | 100 mM |
| Pyrogallol red | 0.068 mM | 0.068 mM | 0.068 mM |
| Ammonium molybdate | 0.047 mM | 0.047 mM | 0.047 mM |
| D-Mannitol | 13.47 mM | 13.47 mM | 13.47 mM |
| Sulfanilic acid | 0.81 mM | 0.81 mM | 0.81 mM |
| Sodium benzoate | 12.65 mM | 12.65 mM | 12.65 mM |
| EDTA-OH | 0.04% | 0.04% | 0.04% |
| SDS | - | - | - |
| n-Octylamine hydrochloride | 0.002% | - | - |
| Benzylcetyldimethylammonium chloride | - | 0.002% | - |
| Lauryldimethylaminoacetic acid betaine | - | - | 0.002% |
| Brij58 | 0.008% | 0.008% | 0.008% |

**[Table 3]**

| | Example 3 | Example 4 | Example 5 |
|---|---|---|---|
| Glycine | 100 mM | 100 mM | 100 mM |
| Pyrogallol red | 0.068 mM | 0.068 mM | 0.068 mM |
| Ammonium molybdate | 0.047 mM | 0.047 mM | 0.047 mM |
| D-Mannitol | 13.47 mM | 13.47 mM | 13.47 mM |
| Sulfanilic acid | 0.81 mM | 0.81 mM | 0.81 mM |
| Sodium benzoate | 12.65 mM | 12.65 mM | 12.65 mM |
| EDTA-OH | 0.04% | 0.04% | 0.04% |
| SDS | 0.002% | 0.002% | 0.002% |
| Brij35 | 0.008% | - | - |
| NONION E-230 | - | 0.008% | - |
| NONION S15.4 | - | - | 0.008% |

**[Table 4]**

| | Comparative Example 8 | Comparative Example 9 | Comparative Example 10 |
|---|---|---|---|
| Glycine | 100 mM | 100 mM | 100 mM |
| Pyrogallol red | 0.068 mM | 0.068 mM | 0.068 mM |
| Ammonium molybdate | 0.047 mM | 0.047 mM | 0.047 mM |
| D-Mannitol | 13.47 mM | 13.47 mM | 13.47 mM |
| Sulfanilic acid | 0.81 mM | 0.81 mM | 0.81 mM |
| Sodium benzoate | 12.65 mM | 12.65 mM | 12.65 mM |
| EDTA-OH | 0.04% | 0.04% | 0.04% |
| SDS | 0.002% | 0.002% | 0.002% |
| TritonX-100 | 0.008% | - | - |
| BPSH-25 | - | 0.008% | - |
| Tween20 | - | - | 0.008% |

### 2-2. Measurement Results

The measurement results are shown below.

**[Table 5]**

| | | Comparative Example 1 | Comparative Example 2 | Comparative Example 3 | Example 1 | Example 2 | Comparative Example 4 |
|---|---|---|---|---|---|---|---|
| Measured value of protein solution mg/dL | | | | | | | |
| | 0 mg/dL Globulin | 0.1 | 0.0 | 0.3 | 0.6 | 1.3 | -0.1 |
| | 120 mg/dL Globulin | 87.6 | 115.5 | 79.1 | 111.2 | 128.4 | 80.7 |
| | 600 mg/dL Globulin | 384.2 | 533.6 | 378.8 | 517.6 | 783.6 | 377.8 |
| | 0 mg/dL Albumin | -0.6 | -1.2 | -0.5 | 0.2 | -0.3 | -0.7 |
| | 120 mg/dL Albumin | 128.9 | 131.7 | 129.7 | 131.1 | 133.3 | 128.4 |
| | 600 mg/dL Albumin | 545.3 | 619.8 | 579.6 | 637.7 | 1012.9 | 554.3 |

| Sensitivity mg/dL | | | | | | | |
|---|---|---|---|---|---|---|---|
| | 120 mg/dL Globulin | 88 | 116 | 79 | 111 | 0.6 | 81 |
| | 600 mg/dL Globulin | 384 | 534 | 378 | 517 | 782 | 378 |
| | 120 mg/dL Albumin | 129 | 133 | 130 | 131 | 134 | 129 |
| | 600 mg/dL Albumin | 546 | 621 | 580 | 638 | 1013 | 555 |

| Globulin sensitivity/albumin sensitivity | | | | | | | |
|---|---|---|---|---|---|---|---|
| | 120 mg/dL | 68% | 87% | 61% | 84% | 95% | 63% |
| | 600 mg/dL | 70% | 86% | 65% | 81% | 77% | 68% |

| Measured value of hemoglobin sample mg/dL | | | | | | | |
|---|---|---|---|---|---|---|---|
| | 0 mg/dL Hemoglobin | 43.7 | 46.3 | 48.0 | 44.4 | 38.0 | 44.2 |
| | 100 mg/dL Hemoglobin | 162.6 | 196.7 | 170.2 | 61.0 | 66.4 | 164.5 |
| Degree of influence 100 mg /dL | | 372% | 424% | 355% | 137% | 175% | 372% |

The sensitivity was calculated by subtracting the measured value of blank (0 mg/dL) from the measured value of each protein solution.

**[Table 6]**

| | | Comparative Example 1 | Example 1 | Comparative Example 5 | Comparative Example 6 | Comparative Example 7 |
|---|---|---|---|---|---|---|
| Measured value of protein solution mg/dL | | | | | | |
| | 0 mg/dL Globulin | 0.2 | 0.3 | 0.1 | -6.1 | 0.0 |
| | 120 mg/dL Globulin | 87.3 | 113.2 | 81.4 | 85.3 | 78.6 |
| | 600 mg/dL Globulin | 374.1 | 522.9 | 372.4 | 353.8 | 357.2 |
| | 0 mg/dL Albumin | -0.2 | -0.6 | -0.2 | -11.3 | -1.4 |
| | 120 mg/dL Albumin | 125.3 | 127.8 | 127.0 | 120.0 | 125.0 |
| | 600 mg/dL Albumin | 528.0 | 613.5 | 541.0 | 436.7 | 527.7 |

| Sensitivity mg/dL | | | | | | |
|---|---|---|---|---|---|---|
| | 120 mg/dL Globulin | 87 | 113 | 81 | 91 | 79 |
| | 600 mg/dL Globulin | 374 | 523 | 372 | 360 | 357 |
| | 120 mg/dL Albumin | 126 | 128 | 127 | 131 | 126 |
| | 600 mg/dL Albumin | 528 | 614 | 541 | 448 | 529 |

| Globulin sensitivity/albumin sensitivity | | | | | | |
|---|---|---|---|---|---|---|
| | 120 mg/dL | 69% | 88% | 64% | 70% | 62% |
| | 600 mg/dL | 71% | 85% | 69% | 80% | 68% |

| Measured value of hemoglobin sample mg/dL | | | | | | |
|---|---|---|---|---|---|---|
| | 0 mg/dL Hemoglobin | 47.7 | 46.5 | 48.3 | 40.1 | 48.2 |
| | 100 mg/dL Hemoglobin | 169.8 | 66.7 | 178.0 | 170.8 | 173.8 |
| Degree of influence 100 mg /dL/0 mg/dL | | 356% | 143% | 369% | 426% | 360% |

The sensitivity was calculated by subtracting the measured value of blank (0 mg/dL) from the measured value of each protein solution.

The numerical values of Comparative Example 1 and Example 1 are different from those in Table 5. This is because the reagents having the same compositions were measured again together with the reagents of Comparative Example 5 and the like.

**[Table 7]**

| | | Example 3 | Example 4 | Example 5 |
|---|---|---|---|---|
| Measured value of protein solution mg/dL | | | | |
| | 0 mg/dL Globulin | -0.8 | 0.4 | 0.1 |
| | 120 mg/dL Globulin | 114.6 | 133.8 | 115.5 |
| | 600 mg/dL Globulin | 529.8 | 632.2 | 537.5 |
| | 0 mg/dL Albumin | -0.8 | -1.1 | -1.1 |
| | 120 mg/dL Albumin | 134.3 | 130.2 | 133.1 |
| | 600 mg/dL Albumin | 636.9 | 651.3 | 654.2 |

| Sensitivity mg/dL | | | | |
|---|---|---|---|---|
| | 120 mg/dL Globulin | 115 | 133 | 115 |
| | 600 mg/dL Globulin | 531 | 632 | 537 |
| | 120 mg/dL Globulin | 135 | 131 | 134 |
| | 600 mg/dL Globulin | 638 | 652 | 655 |

| Globulin sensitivity/albumin sensitivity | | | | |
|---|---|---|---|---|
| | 120 mg/dL | 85% | 102% | 86% |
| | 600 mg/dL | 83% | 97% | 82% |

| Measured value of hemoglobin sample mg/dL | | | | |
|---|---|---|---|---|
| | 0 mg/dL Hemoglobin | 45.9 | 35.8 | 43.9 |
| | 100 mg/dL Hemoglobin | 70.7 | 60.2 | 87.3 |
| Degree of influence 100 mg/dL/0 mg/dL | | 154% | 168% | 199% |

The sensitivity was calculated by subtracting the measured value of blank (0 mg/dL) from the measured value of each protein solution.

**[Table 8]**

| | | Comparative Example 8 | Comparative Example 9 | Comparative Example 10 |
|---|---|---|---|---|
| Measured value of protein solution mg/dL | | | | |
| | 0 mg/dL Globulin | 0.6 | 0.7 | -0.4 |
| | 120 mg/dL Globulin | 100.5 | 191.4 | 108.8 |
| | 600 mg/dL Globulin | 506.2 | 892.7 | 508.2 |
| | 0 mg/dL Albumin | -0.7 | -1.7 | -0.5 |
| | 120 mg/dL Albumin | 128.6 | 131.1 | 126.0 |
| | 600 mg/dL Albumin | 643.5 | 756.9 | 631.5 |

| Sensitivity mg/dL | | | | |
|---|---|---|---|---|
| | 120 mg/dL Globulin | 100 | 191 | 109 |
| | 600 mg/dL Globulin | 506 | 892 | 509 |
| | 120 mg/dL Albumin | 129 | 133 | 126 |
| | 600 mg/dL Albumin | 644 | 759 | 632 |

| Globulin sensitivity/albumin sensitivity | | | | |
|---|---|---|---|---|
| | 120 mg/dL | 77% | 144% | 86% |
| | 600 mg/dL | 78% | 118% | 80% |

| Measured value of hemoglobin sample mg/dL | | | | |
|---|---|---|---|---|
| | 0 mg/dL Hemoglobin | 44.3 | 30.0 | 43.7 |
| | 100 mg/dL Hemoglobin | 171.0 | 74.4 | 180.8 |
| Degree of influence 100 mg/dL/0 mg/dL | | 386% | 248% | 414% |

The sensitivity was calculated by subtracting the measured value of blank (0 mg/dL) from the measured value of each protein solution.

As shown in the above table, the reagent to which SDS and Brij 58 were added (Example 1), the reagent to which sodium linear alkylbenzene sulfonate and Brij 58 were added (Example 2), the reagent to which SDS and Brij 35 were added (Example 3), the reagent to which SDS and NONION E-230 were added (Example 4), and the reagent to which SDS and NONION S15.4 were added (Example 5) significantly reduced the difference in reactivity between protein species (globulin sensitivity/albumin sensitivity), and the degree of influence of hemolytic hemoglobin, as compared with the reagent containing no surfactant (Comparative Example 1) and the reagent to which only Brij 58 was added (Comparative Example 3). As compared with the reagent to which only SDS was added (Comparative Example 2), the difference in reactivity between protein species (globulin sensitivity/albumin sensitivity) was comparable or reduced, and the degree of influence of hemolytic hemoglobin was significantly reduced.

In addition, the reagents to which Brij 58, and sodium cholate, n-octylamine hydrochloride, benzylcetyldimethylammonium chloride or lauryldimethylaminoacetic acid betaine were added (Comparative Examples 4 to 7) did not much improve either the difference in reactivity between protein species (globulin sensitivity/albumin sensitivity) or the influence of hemolytic hemoglobin.

Furthermore, the reagents to which SDS, and Triton X-100, BPSH-25 or Tween 20 were added (Comparative Examples 8 to 10) improved the difference in reactivity between protein species (globulin sensitivity/albumin sensitivity), but did not much improved the influence of hemolytic hemoglobin.

### 3. Study on Concentration of Polyoxyalkylene-Type Nonionic Surfactant

### 3-1. Preparation of Reagents

### [Examples 6 to 8]

Measurement reagents were each prepared in the same manner as in Example 1 except that the concentration of Brij 58 was changed to 0.002%, 0.004%, or 0.016%.

The compositions of the measurement reagents of Examples 6 to 8 are collectively shown below together with the measurement reagents of Example 1 and Comparative Examples 1 and 2.

**[Table 9]**

| | Comparative Example 1 | Comparative Example 2 | Example 6 | Example 7 | Example 1 | Example 8 |
|---|---|---|---|---|---|---|
| Glycine | 100 mM | 100 mM | 100 mM | 100 mM | 100 mM | 100 mM |
| Pyrogallol red | 0.068 mM | 0.068 mM | 0.068 mM | 0.068 mM | 0.068 mM | 0.068 mM |
| Ammonium molybdate | 0.047 mM | 0.047 mM | 0.047 mM | 0.047 mM | 0.047 mM | 0.047 mM |
| D-Mannitol | 13.47 mM | 13.47 mM | 13.47 mM | 13.47 mM | 13.47 mM | 13.47 mM |
| Sulfanilic acid | 0.81 mM | 0.81 mM | 0.81 mM | 0.81 mM | 0.81 mM | 0.81 mM |
| Sodium benzoate | 12.65 mM | 12.65 mM | 12.65 mM | 12.65 mM | 12.65 mM | 12.65 mM |
| EDTA-OH | 0.04% | 0.04% | 0.04% | 0.04% | 0.04% | 0.04% |
| SDS | - | 0.002% | 0.002% | 0.002% | 0.002% | 0.002% |
| Brij58 | - | - | 0.002% | 0.004% | 0.008% | 0.016% |

### 3-2. Measurement Results

The results of testing the reagents of Examples 1 and 7 to 11 and Comparative Example 1 for their effects of improving the reactivity difference between albumin and γ-globulin and the hemolytic hemoglobin interference are summarized in the following table together with the results of the reagent of Comparative Example 2 shown in Table 5.

**[Table 10]**

| | | Comparative Example 1 | Comparative Example 2 | Example 6 | Example 7 | Example 1 | Example 8 |
|---|---|---|---|---|---|---|---|
| Measured value of protein solution mg/dL | | | | | | | |
| | 0 mg/dL Globulin | -0.2 | 0.0 | 0.0 | -0.3 | 0.1 | -0.2 |
| | 120 mg/dL Globulin | 85.5 | 115.5 | 129.3 | 119.5 | 109.7 | 97.3 |
| | 600 mg/dL Globulin | 381.2 | 533.6 | 597.8 | 557.6 | 513.2 | 457.3 |
| | 0 mg/dL Albumin | -1.0 | -1.2 | -1.5 | -1.2 | -1.0 | -0.9 |
| | 120 mg/dL Albumin | 128.8 | 131.7 | 131.6 | 133.0 | 129.4 | 131.2 |
| | 600 mg/dL Albumin | 546.0 | 619.8 | 691.8 | 651.4 | 614.3 | 588.8 |
| Sensitivity mg/dL | | | | | | | |
| | 120 mg/dL Globulin | 86 | 116 | 129 | 120 | 110 | 97 |
| | 600 mg/dL Globulin | 381 | 534 | 598 | 558 | 513 | 457 |
| | 120 mg/dL Albumin | 130 | 133 | 133 | 134 | 130 | 132 |
| | 600 mg/dL Albumin | 547 | 621 | 693 | 653 | 615 | 590 |
| Globulin sensitivity/albumin sensitivity | | | | | | | |
| | 120 mg/dL | 66% | 87% | 97% | 89% | 84% | 74% |
| | 600 mg/dL | 70% | 86% | 86% | 86% | 83% | 78% |
| Measured value of hemoglobin sample mg/dL | | | | | | | |
| | 0 mg/dL Hemoglobin | 45.7 | 46.3 | 41.2 | 43.9 | 44.0 | 48.1 |
| | 100 mg/dL Hemoglobin | 168.1 | 196.7 | 102.2 | 68.1 | 63.8 | 73.6 |
| Degree of influence 100 mg /dL/0 mg/dL | | 368% | 424% | 248% | 155% | 145% | 153% |

The sensitivity was calculated by subtracting the measured value of blank (0 mg/dL) from the measured value of each protein solution.

The numerical values of Comparative Example 1 and Example 1 are different from those in Tables 5 and 6. This is because the reagents having the same compositions were measured again together with the reagents of Comparative Example 7 and the like.

As shown in the above table, in cases where Brij 58 was 0.002% by mass to 0.016% by mass (Examples 6, 7, 1, and 8), both the reactivity difference between protein species and the influence of hemolytic hemoglobin were improved.

### 4. Study on Concentration of Sulfonic Acid-Type Anionic Surfactant or Sulfuric Acid Ester-Type Anionic Surfactant

### 4-1. Preparation of Reagents

### [Examples 9 to 11]

Measurement reagents were each prepared in the same manner as in Example 1 except that the concentration of Na dodecyl sulfate (SDS) was changed to 0.001%, 0.003%, or 0.0035%.

The compositions of the measurement reagents of Examples 9 to 11 are collectively shown below together with those of the measurement reagents of Example 1 and Comparative Examples 1 and 3.

**[Table 11]**

| | Comparative Example 1 | Comparative Example 3 | Example 9 | Example 1 | Example 10 | Example 11 |
|---|---|---|---|---|---|---|
| Glycine | 100 mM | 100 mM | 100 mM | 100 mM | 100 mM | 100 mM |
| Pyrogallol red | 0.068 mM | 0.068 mM | 0.068 mM | 0.068 mM | 0.068 mM | 0.068 mM |
| Ammonium molybdate | 0.047 mM | 0.047 mM | 0.047 mM | 0.047 mM | 0.047 mM | 0.047 mM |
| D-Mannitol | 13.47 mM | 13.47 mM | 13.47 mM | 13.47 mM | 13.47 mM | 13.47 mM |
| Sulfanilic acid | 0.81 mM | 0.81 mM | 0.81 mM | 0.81 mM | 0.81 mM | 0.81 mM |
| Sodium benzoate | 12.65 mM | 12.65 mM | 12.65 mM | 12.65 mM | 12.65 mM | 12.65 mM |
| EDTA-OH | 0.04% | 0.04% | 0.04% | 0.04% | 0.04% | 0.04% |
| SDS | - | - | 0.001% | 0.002% | 0.003% | 0.0035% |
| Brij58 | - | 0.008% | 0.008% | 0.008% | 0.008% | 0.008% |

### 4-2. Measurement Results

The results of testing the reagents of Examples 1 and 9 to 11 and Comparative Examples 1 and 3 for the effects of improving the reactivity difference between albumin and γ-globulin and the hemolytic hemoglobin interference are summarized in the following table.

**[Table 12]**

| | | Comparative Example 1 | Comparative Example 3 | Example 9 | Example 1 | Example 10 | Example 11 |
|---|---|---|---|---|---|---|---|
| Measured value of protein solution mg/dL | | | | | | | |
| | 0 mg/dL Globulin | -0.2 | 0.8 | 0.6 | 1.0 | 1.8 | 1.0 |
| | 120 mg/dL Globulin | 85.5 | 80.5 | 101.3 | 111.9 | 159.7 | 189.7 |
| | 600 mg/dL Globulin | 381.2 | 379.0 | 465.9 | 514.7 | 716.5 | 874.1 |
| | 0 mg/dL Albumin | -1.0 | 0.5 | 0.9 | 0.8 | 2.5 | 2.0 |
| | 120 mg/dL Albumin | 128.8 | 129.8 | 130.0 | 129.3 | 134.1 | 130.1 |
| | 600 mg/dL Albumin | 546.0 | 570.8 | 614.3 | 617.6 | 700.0 | 779.1 |
| Sensitivity mg/dL | | | | | | | |
| | 120 mg/dL Globulin | 86 | 80 | 101 | 111 | 158 | 189 |
| | 600 mg/dL Globulin | 381 | 378 | 465 | 514 | 715 | 873 |
| | 120 mg/dL Albumin | 130 | 129 | 129 | 128 | 132 | 128 |
| | 600 mg/dL Albumin | 547 | 570 | 613 | 617 | 698 | 777 |
| Globulin sensitivity/albumin sensitivity | | | | | | | |
| | 120 mg/dL | 66% | 62% | 78% | 86% | 120% | 147% |
| | 600 mg/dL | 70% | 66% | 76% | 83% | 102% | 112% |
| Measured value of hemoglobin sample mg/dL | | | | | | | |
| | 0 mg/dL Hemoglobin | 45.7 | 48.4 | 48.0 | 43.7 | 27.9 | 22.0 |
| | 100 mg/dL Hemoglobin | 168.1 | 171.0 | 82.9 | 62.2 | 47.9 | 37.2 |
| Degree of influence 100 mg /dL/0 mg/dL | | 368% | 353% | 173% | 142% | 172% | 169% |

The sensitivity was calculated by subtracting the measured value of blank (0 mg/dL) from the measured value of each protein solution.

The numerical values of Comparative Examples 1 and 3 and Example 1 are different from those in Tables 5 and 6. This is because the reagents having the same compositions were measured again together with the reagents of Example 9 and the like.

As shown in the above table, in cases where the SDS was 0.001% by mass to 0.0035% by mass (Examples 9, 1, 10, and 11), both the reactivity difference between protein species and the influence of hemolytic hemoglobin were improved.

## Claims

1. A method for quantifying protein in urine using a complex in which a metal is coordinated to a dye and an absorption wavelength of which shifts upon binding to the protein, the method comprising:
reacting the protein with the complex in presence of
at least one anionic surfactant selected from a sulfonic acid-type anionic surfactant and a sulfuric acid ester-type anionic surfactant; and
a polyoxyalkylene-type nonionic surfactant.

2. A method for reducing hemolytic hemoglobin interference and reducing a reactivity difference between protein species in quantification of protein in urine using a complex in which a metal is coordinated to a dye and an absorption wavelength of which shifts upon binding of the protein, the method comprising:
reacting the protein with the complex in presence of
at least one anionic surfactant selected from a sulfonic acid-type anionic surfactant and a sulfuric acid ester-type anionic surfactant; and
a polyoxyalkylene-type nonionic surfactant.

3. The method according to claim 1 or 2, wherein
the sulfonic acid-type anionic surfactant is a sulfonate represented by Formula (1):
R¹⁻SO₃X¹··· (1)
wherein
R¹ is selected from the group consisting of:
(1) a hydrocarbon group having 8 to 18 carbon atoms,
(2) a monocyclic or bicyclic aromatic hydrocarbon substituted with a hydrocarbon group having 8 to 18 carbon atoms,
(3) a succinic acid residue of Formula (2), a (di)ester of the succinic acid residue, or a salt of the succinic acid residue or the (di)ester: 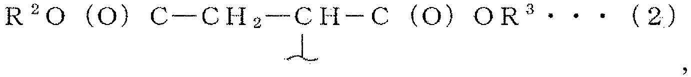 wherein R² and R³ each independently represent hydrogen, a hydrocarbon group having 8 to 18 carbon atoms, or an alkali metal,
(4) a succinic acid monoamide residue of Formula (3), an ester of the succinic acid monoamide residue, or a salt of the succinic acid monoamide residue or the ester: 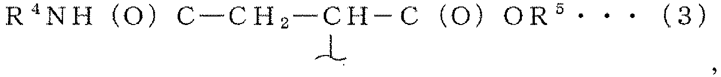 wherein R⁴ and R⁵ each independently represent hydrogen, a hydrocarbon group having 8 to 18 carbon atoms, or an alkali metal, and
(5) a sulfonate of a hydrocarbon group-substituted diphenyl ether residue of Formula (4): 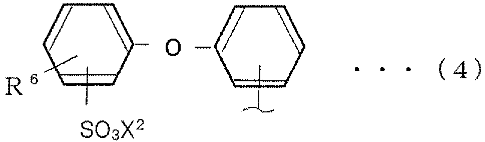
wherein R⁶ is a hydrocarbon group having 8 to 18 carbon atoms, and X² is an alkali metal, ammonium, or alkanolamine, and
X¹ is an alkali metal, ammonium, or alkanolamine.

4. The method according to claim 3, wherein R¹ is (1) an alkyl group or alkenyl group having 8 to 18 carbon atoms or (2) a monocyclic or bicyclic aromatic hydrocarbon substituted with an alkyl group or alkenyl group having 8 to 18 carbon atoms.

5. The method according to claim 4, wherein the sulfonic acid-type anionic surfactant is an alkane sulfonate having 11 to 16 carbon atoms or a linear alkylbenzene sulfonate having 10 to 14 carbon atoms.

6. The method according to claim 1 or 2, wherein the sulfuric acid ester-type anionic surfactant is a sulfuric acid ester represented by Formula (5) or (6):
R⁷O-SO₃X³··· (5)
wherein R⁷ is a hydrocarbon group having 8 to 18 carbon atoms or a monocyclic or bicyclic aromatic hydrocarbon substituted with a hydrocarbon group having 8 to 18 carbon atoms, and X³ is an alkali metal, ammonium, or alkanolamine, or
R⁸O-(CHR⁹CHR¹⁰O)ₙ-SO₃X⁴··· (6)
wherein R⁸ is a hydrocarbon group having 8 to 18 carbon atoms, R⁹ and R¹⁰ are independently hydrogen or an alkyl group having 1 to 3 carbon atoms, n is 1 to 4, and X⁴ is an alkali metal, ammonium, or alkanolamine.

7. The method according to claim 6, wherein the sulfuric acid ester-type anionic surfactant is an alkyl sulfate having 11 to 16 carbon atoms.

8. The method according to any one of claims 1 to 7, wherein the polyoxyalkylene-type nonionic surfactant is a polyoxyalkylene compound of Formula (7) or (8):
R¹¹-Y¹-((CH₂)ₘO)ₙ-R¹²··· (7)
wherein Y¹ is O, C(O)O, or C(O)NH, R¹¹ is a chain hydrocarbon group having 8 to 22 carbon atoms, m is an integer of 1 to 4, n is an integer of 2 to 35, and R¹² is hydrogen or an alkyl group having 1 to 3 carbon atoms,
R¹³-Y²-((CH₂)ₘ₁O)ₙ₁-((CH₂)ₘ₂O)ₙ₂-((CH₂)ₘ₃O)ₙ₃-R¹⁴··· (8)
wherein Y² is O, C(O)O, or C(O)NH, m1 and m3 are each independently an integer of 1 to 4, m2 is an integer of 2 to 4, m1 and/or m3 is different from m2, n1, n2, and n3 are each independently an integer of 2 to 200, R¹³ is hydrogen or a chain hydrocarbon group having 1 to 30 carbon atoms, and R¹⁴ is hydrogen or an alkyl group having 1 to 3 carbon atoms.

9. The method according to claim 8, wherein the polyoxyalkylene-type nonionic surfactant is a polyoxyalkylene compound of Formula (7) wherein Y¹ is O or C(O)O, R¹¹ is a linear alkyl group or alkenyl group having 11 to 20 carbon atoms, m is 2, n is an integer of 20 to 35, and R¹² is hydrogen.

10. The method according to any one of claims 1 to 9, wherein the nonionic surfactant in a reaction solution has a concentration of 0.0015% by mass to 0.03% by mass, and the anionic surfactant in the reaction solution has a concentration of 0.001% by mass to 0.0035% by mass.

11. The method according to claim 10, wherein the anionic surfactant in the reaction solution has a concentration of 0.0015% by mass to 0.003% by mass.

12. The method according to claim 10 or 11, wherein the nonionic surfactant in the reaction solution has a concentration of 0.0025% by mass to 0.024% by mass.

13. The method according to any one of claims 1 to 12, wherein the dye is pyrogallol red or pyrocatechol violet, and the metal is molybdenum, tin, or iron.

14. A reagent for measuring protein in urine comprising:
a complex in which a metal is coordinated to a dye and an absorption wavelength of which shifts upon binding to the protein;
at least one anionic surfactant selected from a sulfonic acid-type anionic surfactant and a sulfuric acid ester-type anionic surfactant; and
a polyoxyalkylene-type nonionic surfactant.

15. The reagent for measuring protein in urine according to claim 14, wherein
the sulfonic acid-type anionic surfactant is a sulfonate represented by Formula (1):
R¹SO₃X¹··· (1)
wherein
R¹ is selected from the group consisting of:
(1) a hydrocarbon group having 8 to 18 carbon atoms,
(2) a monocyclic or bicyclic aromatic hydrocarbon substituted with a hydrocarbon group having 8 to 18 carbon atoms,
(3) a succinic acid residue of Formula (2), a (di)ester of the succinic acid residue, or a salt of the succinic acid residue or the (di)ester: 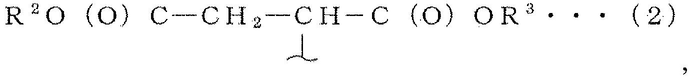 wherein R² and R³ each independently represent hydrogen, a hydrocarbon group having 8 to 18 carbon atoms, or an alkali metal,
(4) a succinic acid monoamide residue of Formula (3), an ester of the succinic acid monoamide residue, or a salt of the succinic acid monoamide residue or the ester: 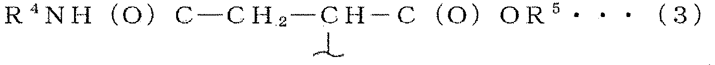 wherein R⁴ and R⁵ each independently represent hydrogen, a hydrocarbon group having 8 to 18 carbon atoms, or an alkali metal, and
(5) a sulfonate of a hydrocarbon group-substituted diphenyl ether residue, of Formula (4): 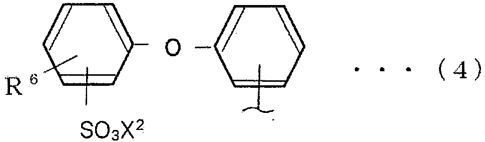
wherein R⁶ is a hydrocarbon group having 8 to 18 carbon atoms, and X² is an alkali metal, ammonium, or alkanolamine, and
X¹ is an alkali metal, ammonium, or alkanolamine.

16. The reagent for measuring protein in urine according to claim 15, wherein R¹ is (1) an alkyl group or alkenyl group having 8 to 18 carbon atoms, or (2) a monocyclic or bicyclic aromatic hydrocarbon substituted with an alkyl group or alkenyl group having 8 to 18 carbon atoms.

17. The reagent for measuring protein in urine according to claim 16, wherein the sulfonic acid-type anionic surfactant is an alkane sulfonate having 11 to 16 carbon atoms or a linear alkylbenzene sulfonate having 10 to 14 carbon atoms.

18. The reagent for measuring protein in urine according to claim 14, wherein the sulfuric acid ester-type anionic surfactant is a sulfuric acid ester represented by Formula (5) or (6):
R⁷O-SO₃X³··· (5)
wherein R⁷ is a hydrocarbon group having 8 to 18 carbon atoms or a monocyclic or bicyclic aromatic hydrocarbon substituted with a hydrocarbon group having 8 to 18 carbon atoms, and X³ is an alkali metal, ammonium, or alkanolamine, or
R⁸O-(CHR⁹CHR¹⁰O)ₙ-SO₃X⁴··· (6)
wherein R⁸ is a hydrocarbon group having 8 to 18 carbon atoms, R⁹ and R¹⁰ are independently hydrogen or an alkyl group having 1 to 3 carbon atoms, n is 1 to 4, and X⁴ is an alkali metal, ammonium, or alkanolamine.

19. The reagent for measuring protein in urine according to claim 18, wherein the sulfuric acid ester-type anionic surfactant is an alkyl sulfate having 11 to 16 carbon atoms.

20. The reagent for measuring protein in urine according to any one of claims 14 to 19, wherein the polyoxyalkylene-type nonionic surfactant is a polyoxyalkylene compound of Formula (7) or (8):
R¹¹-Y¹-((CH₂)ₘO)ₙ-R¹²··· (7)
wherein Y' is O, C(O)O, or C(O)NH, R¹¹ is a chain hydrocarbon group having 8 to 22 carbon atoms, m is an integer of 1 to 4, n is an integer of 2 to 35, and R¹² is hydrogen or an alkyl group having 1 to 3 carbon atoms,
R¹³-Y²-((CH₂)ₘ₁O)ₙ₁-((CH₂)ₘ₂O)ₙ₂-((CH₂)ₘ₃O)ₙ₃-R¹⁴··· (8)
wherein Y² is O, C(O)O, or C(O)NH, m1 and m3 are each independently an integer of 1 to 4, m2 is an integer of 2 to 4, m1 and/or m3 is different from m2, n1, n2, and n3 are each independently an integer of 2 to 200, R¹³ is hydrogen or a chain hydrocarbon group having 1 to 30 carbon atoms, and R¹⁴ is hydrogen or an alkyl group having 1 to 3 carbon atoms.

21. The reagent for measuring protein in urine according to claim 20, wherein the polyoxyalkylene-type nonionic surfactant is a polyoxyalkylene compound of Formula (7) wherein Y¹ is O or C(O)O, R¹¹ is a linear alkyl group or alkenyl group having 11 to 20 carbon atoms, m is 2, n is an integer of 20 to 35, and R¹² is hydrogen.

22. The reagent for measuring protein in urine according to any one of claims 14 to 21, wherein the nonionic surfactant has a concentration of 0.0015% by mass to 0.03% by mass, and the anionic surfactant has a concentration of 0.001% by mass to 0.0035% by mass.

23. The reagent for measuring protein in urine according to claim 22, wherein the anionic surfactant has a concentration of 0.0015% by mass to 0.003% by mass.

24. The reagent for measuring protein in urine according to claim 22 or 23, wherein the nonionic surfactant has a concentration of 0.0025% by mass to 0.024% by mass.

25. The reagent for measuring protein in urine according to any one of claims 14 to 24, wherein the dye is pyrogallol red or pyrocatechol violet, and the metal is molybdenum, tin, or iron.

26. The reagent for measuring protein in urine according to any one of claims 14 to 25, which is a single-solution type reagent for measurement.

27. A method for producing a reagent for measuring protein in urine, comprising dissolving, in a buffer solution,
a dye selected from pyrocatechol violet, pyrogallol red, brompyrogallol red, o-hydroxyhydroquinonephthalein, and gallein;
an oxyacid salt, a halide, a complex salt, or an organic or inorganic acid salt of a metal selected from molybdenum, tin, and iron;
at least one anionic surfactant selected from a sulfonic acid-type anionic surfactant and a sulfuric acid ester-type anionic surfactant; and
a polyoxyalkylene-type nonionic surfactant.
